# EUROPEAN PATENT APPLICATION

(11) **EP 4 696 783 A1**
(43) Date of publication of application: **18.02.2026**
(21) Application number: 24788851.4
(22) Date of filing: 12.04.2024
(51) Int. Cl.: C12P 1/04, C07K 1/34, C12N 1/21, C12N 15/31

(54) **METHOD FOR IMPROVING FILTRATION SPEED**

(30) Priority: 13.04.2023 JP 2023065982
(71) Applicant: Kao Corporation, Tokyo 103-8210 (JP)
(72) Inventor: HONJO, Hiroshi, Tokyo 103-8210 (JP); MASUDA, Kenta, Tokyo 103-8210 (JP); KAWAHARA, Akihito, Tokyo 103-8210 (JP); KIMURA, Shunsuke, Tokyo 103-8210 (JP); SHIMIZU, Yosuke, Tokyo 103-8210 (JP)
(74) Representative: Vossius & Partner Patentanwälte Rechtsanwälte mbB
(86) International application number: PCT/JP2024/014879
(87) International publication number: WO 2024/214829

(57) **Abstract**

Provided is a method for improving the filtration rate in the microfiltration step when producing a useful substance using a microorganism.

A method for improving the filtration rate in the microfiltration step of the fermentation production of a useful substance using a microorganism, wherein a modified microorganism, in which the function of a flagellar gene of the microorganism is suppressed, is used as the microorganism.

## Description

### Field of the Invention

The present invention relates to a method for improving the filtration rate of microfiltration in the fermentation production of a useful substance.

### Background of the Invention

In the production of useful substances using microorganisms, a step of filtering a fermentation solution to remove insoluble substances such as fine particles, cultured cells, and spores in the fermentation solution is required to recover the target fermentation product such as an enzyme.

However, in the filtration process, particles with sizes close to that of the separation membrane pores may cause clogging of the separation membrane, and furthermore, insoluble substances may deposit on the membrane surface, which can lead to a decrease in filtration efficiency.

Heretofore, with regards to such physical clogging of the separation membrane and deposition of insoluble substances on the membrane surface, procedures such as sweeping the membrane surface while filtering are used to avoid clogging and deposition of insoluble substances. However, in the case of fermentation solutions containing high concentrations of microbial cells, the viscosity of the fermentation solution is high, resulting in a significant pressure drop during sweeping and an increase in filtration pressure, which causes the compaction of insoluble substances on the membrane surface, and ultimately leads to a decrease in filtration efficiency.

To improve the permeation speed in microfiltration, solutions involving processes such as sweeping of insoluble substances on the membrane surface and solutions using additives such as cationic surfactants, have also been used. For example, Patent Literature 1 discloses that, in the microfiltration, the addition of 0.01 to 1 (w/v) of a cationic surfactant to an enzyme-producing fermentation culture solution containing a high concentration of microorganisms improves both the permeate flux during microfiltration and filtration efficiency.

However, these solutions have problems such as an increased complexity of the fermentation process, the prolonged production time due to the increased complexity, and contamination of the final formulation with additives.

Patent Literature 1: JP-B-4808725

### Disclosure of the Invention

The present invention relates to the following.

A method for improving the filtration rate in the microfiltration step of the fermentation production of a useful substance using a microorganism, wherein a modified microorganism, in which the function of a flagellar gene of the microorganism is suppressed, is used as the microorganism.

### Brief Description of Drawings

Figure 1 illustrates the introduction of a *prsA* gene into the *nprE* locus of a *ΔsigF* strain by homologous recombination.
Figure 2 illustrates the deletion of the ORF sequence of the hag gene by homologous recombination.
Figure 3 illustrates the change in cell density of the culture solution of the modified microorganism of the present invention.
Figure 4 illustrates the effect of the culture solution of the modified microorganism of the present invention in improving the permeate flux during microfiltration.

### Detailed Description of the Invention

The present invention relates to providing a method for improving the filtration rate in the microfiltration step when producing a useful substance using a microorganism.

As a result of studies conducted in view of the above problems, the present inventors found that the use of a microorganism in which the function of a flagellar gene is suppressed improves the filtration rate significantly in microfiltration.

The present invention can improve the filtration rate in the microfiltration step of the fermentation production of a useful substance, thus reducing the time and cost required for producing the useful substance.

The method of the present invention is a method for improving the filtration rate in the microfiltration step in the fermentation production of a useful substance using a microorganism, wherein a modified microorganism in which the function of a flagellar gene of the microorganism is suppressed, is used as the microorganism.

The microorganism serving as the host for the modified microorganism used in the method of the present invention is not limited, as long as it can be used in the fermentation production of a useful substance. Examples thereof include bacteria of the genus *Bacillus,* such as *Bacillus subtilis, Bacillus licheniformis, Bacillus cereus, Bacillus thuringiensis,* and *Bacillus amyloliquefaciens*; bacteria of the genus *Escherichia,* such as *Escherichia coli*; bacteria of the genus *Brevibacillus,* such as *Brevibacillus choshinensis* and *Brevibacillus brevis*; bacteria of the genus *Clostridium,* such as *Clostridium butyricum*; and bacteria of the genus *Corynebacterium,* such as *Corynebacterium glutamicum, Corynebacterium efficiens, Corynebacterium ammoniagenes, Corynebacterium halotolerance, Corynebacterium alkanolyticum, Corynebacterium crenatum, Corynebacterium crudilactis,* and *Corynebacterium callunae*; and yeast. Of these, bacteria of the genus *Bacillus,* the genus *Escherichia,* and the genus *Brevibacillus* are preferred; bacteria of the genus *Bacillus* are more preferred; and *Bacillus subtilis* is even more preferred.

The above host microorganism may be a wild strain, or a mutant strain which has been subjected to mutation. Examples of mutant strains include *Bacillus subtilis* mutant strains which overexpress the gene for *prsA*, which is a protein involved in the protein secretion process (JP-A-2007-49987), and *Bacillus subtilis* mutant strains in which sporulation-related genes (e.g., one or more selected from the group consisting of the *sigE* gene, *sigF* gene, *spoIIE* gene, *spoIISB gene, sigG* gene, gene groups included in the region from *spoIVCB* to *spoIIIC,* and genes equivalent to these genes or gene groups) have been deleted or inactivated (JP-A-2003-47490).

Here, the details of the *sigE* gene, *sigF* gene, *spoIIE* gene, *spoIISB gene, sigG* gene, and the gene groups included in the region from *spoIVCB* to *spoIIIC* in *Bacillus subtilis,* are as shown in Table 1 below. Further, genes derived from other microorganisms, preferably from bacteria of the genus *Bacillus,* which have a nucleotide sequence identity of 70% or more, preferably 80% or more, and more preferably 90% or more with these genes are considered to be genes equivalent to those listed in Table 1, and are included within the sporulation-related genes.

**[Table 1]**

| Gene | Location (kb) | Function |
|---|---|---|
| sigE | 1604 | Stage II, mother cell-specific factor σE |
| sigF | 2443 | Stage II, forespore-specific factor σF |
| spoIISB | 1328 | Stage II onward, involved in sporulation |
| spoIIE | 71 | Stage II, activation of forespore-specific factor σF |
| sigG | 1605 | Stages III-V, forespore-specific factor σG |
| spoIVCB- spoIIIC | 2652-2701 | Stages IV-V, mother cell-specific factor σK |

The term "flagellar gene" refers to a gene encoding a flagellin protein of a microorganism.

The flagellar gene is referred to as the hag gene in bacteria of the genus *Bacillus,* the *fliC* gene in *Escherichia coli,* and the *flaA* gene in *Clostridium butyricum.*

For example, the *hag* gene of *Bacillus subtilis* is a gene encoding a protein consisting of an amino acid sequence set forth in SEQ ID NO: 2, and examples thereof include a polynucleotide consisting of a nucleotide sequence set forth in SEQ ID NO: 1. The *hag* gene of *Bacillus licheniformis* is a gene encoding a protein consisting of an amino acid sequence set forth in SEQ ID NO: 4, and examples thereof include a polynucleotide consisting of a nucleotide sequence set forth in SEQ ID NO: 3. The *hag* gene of *Brevibacillus brevis* is a gene encoding a protein consisting of an amino acid sequence set forth in SEQ ID NO: 6, and examples thereof include a polynucleotide consisting of a nucleotide sequence set forth in SEQ ID NO: 5. The *fliC* gene of *Escherichia coli* is a gene encoding a protein consisting of an amino acid sequence set forth in SEQ ID NO: 8, and examples thereof include a polynucleotide consisting of a nucleotide sequence set forth in SEQ ID NO: 7. The flaA gene of *Clostridium butyricum* is a gene encoding a protein consisting of an amino acid sequence set forth in SEQ ID NO: 10, and examples thereof include a polynucleotide set forth in SEQ ID NO: 9.

The flagellar gene of the present invention includes polynucleotides encoding polypeptides which are functionally equivalent to flagellin proteins (e.g., proteins consisting of an amino acid sequence set forth in SEQ ID NOs: 2, 4, 6, 8, or 10) and have an amino acid sequence identity of at least 90% or more, preferably 95% or more, preferably 96% or more, preferably 97% or more, preferably 98% or more, and preferably 99% or more with the flagellin proteins. Here, the functionally equivalent polypeptide refers to a polypeptide exhibiting a biological function, a physiological function, or a biochemical function equivalent to that of the flagellin protein.

This polynucleotide can also be isolated and identified using gene amplification methods based on its nucleotide sequence.

Herein, the identity of an amino acid sequence is calculated by the Lipman-Pearson method (Science, 1985, 227:1435-1441). Specifically, the identity is calculated using the homology analysis (Search homology) program of the genetic information processing software GENETYX Ver. 12, and performing the analysis with the unit size to compare (ktup) set to 2.

The suppression of the function of a flagellar gene includes suppression of the expression of the gene, such as a reduction of the expression levels, repression of transcription, and repression of translation.

Examples of means for reducing the expression levels of a flagellar gene include deletion or inactivation of the gene, specifically, the deletion or inactivation of the coding region, non-coding region, or transcription or translation initiation region of the flagellar gene. Here, examples of inactivation include the introduction of a mutation which reduces the expression levels of the gene.

Examples of means for suppressing transcription of a flagellar gene include introducing a polynucleotide or RNA having the activity of degrading the transcript of the flagellar gene, and examples of means for suppressing translation of a flagellar gene include introducing a polynucleotide or RNA which suppresses the translation of the transcript into the protein.

As one embodiment, the reduction in gene expression levels by deletion or inactivation of the flagellar gene can be achieved by removing all or part of the nucleotide sequence of the flagellar gene from the genome or replacing it with another nucleotide sequence, inserting another polynucleotide fragment into the sequence of the flagellar gene, introducing a mutation into the transcription or translation initiation region of the flagellar gene, or the like, and preferably includes deleting or inactivating all or part of the nucleotide sequence of the flagellar gene. More specific examples include a method of specifically deleting or inactivating a flagellar gene on the genome of a microorganism, and a method of introducing random deletion or random inactivating mutations into the genes within the microorganism cells, and then selecting cells having the desired mutation by evaluation of flagellin protein expression levels or activity or genetic analysis.

Examples of the reduction in gene expression levels by specific deletion or inactivation of a flagellar gene include a method using homologous recombination. That is, it is possible to delete or inactivate a flagellar gene on a genome by constructing a DNA fragment of a flagellar gene into which an inactivating mutation has been introduced via substitution or insertion of a polynucleotide, or a DNA fragment including the flanking region of the flagellar gene but not the flagellar gene itself, and integrating this into the parent microorganism to induce homologous recombination at the region containing the flagellar gene in the genome of the parent microorganism.

Alternatively, it is also possible to inactivate a flagellar gene by introducing, into the parent microorganism, a recombinant vector (such as a plasmid) having a DNA fragment containing a partial region of the flagellar gene to disrupt the partial region of the flagellar gene in the genome of the parent microorganism through homologous recombination.

In particular, when *Bacillus subtilis* is used as the host microorganism, several methods for deleting or inactivating a target gene by homologous recombination have already been reported (such as Mol. Gen. Genet., 223, 268, 1990 and Genet. Syst., 84(4): 315-8, 2009), and the modified microorganism of the present invention can be obtained by using such methods.

Examples of other methods for reducing the gene expression levels by specific deletion or inactivation of a flagellar gene include a method using genome editing. Specific examples of the genome editing techniques include double-strand DNA breaks induced by TALEN or CRISPR-Cas systems and the resulting specific DNA mutations and single-base editing without double-strand DNA breaks.

Examples of methods for reducing the gene expression levels by random deletion or random inactivation of a gene in a microorganism include a method of introducing, into a microorganism, a DNA fragment obtained by randomly cloning a gene into which an inactivating mutation has been introduced, to induce homologous recombination with the gene on the genome of the microorganism, and a method of inducing a mutation by irradiation of the microorganism with ultraviolet rays, gamma rays, or the like. An inactivating mutation of a gene refers to a mutation in which the function originally possessed by the target gene is lost due to a silent mutation, missense mutation, nonsense mutation, frameshift mutation, or the like. For example, a gene into which an inactivating mutation has been introduced either does not express a protein or expresses a protein whose original activity is impaired.

Examples of methods for producing a DNA fragment containing a flagellar gene into which an inactivating mutation has been introduced include site-directed mutagenesis. The site-directed mutagenesis can be performed using mutagenic primers containing the nucleotide mutation to be introduced. For example, it is possible to construct a DNA fragment containing a desired mutation by producing a DNA fragment in which the upstream and downstream sides of the region containing the flagellar gene are amplified by PCR using the flagellar gene as a template with two sets of primers containing the nucleotide mutation to be introduced, and then joining these together by SOE-PCR (splicing by overlap extension PCR) (Gene, 1989, 77(1): pp. 61-68). Alternatively, for site-directed mutagenesis, inverse PCR or annealing methods (Muramatsu et al., New Genetic Engineering Handbook, Revised 4th Edition, Yodosha, pp. 82-88), or commercially available site-directed mutagenesis kits, such as the QuickChange II Site-Directed Mutagenesis Kit or the QuickChange Multi Site-Directed Mutagenesis Kit from Stratagene, can be used.

The mutagenic primers can be produced by known oligonucleotide synthesis methods such as the phosphoramidite method (Nucleic Acids Research, 1989, 17:7059-7071). The flagellar gene as the template may be prepared from the host microorganism by a conventional method or may be chemically synthesized.

To introduce a DNA fragment or vector into a host microorganism, known techniques such as the calcium phosphate method, electroporation, lipofection, gene gun method, and PEG method can be applied. For example, depending on the host microorganism, the competent cell transformation method (J. Bacteriol., 1967, 93:1925-1937), electroporation method (FEMS Microbiol. Lett., 1990, 55:135-138), protoplast transformation method (Mol. Gen. Genet., 1979, 168:111-115), Tris-PEG method (J. Bacteriol., 1983, 156:1130-1134), and the like can be employed.

Examples of polynucleotides having the activity of degrading the transcript of a flagellar gene or polynucleotides suppressing the translation of the transcript into a protein, include polynucleotides containing a nucleotide sequence, or a portion thereof, which is complementary or substantially complementary to the nucleotide sequence of the mRNA of the flagellar gene. Specific examples include the antisense RNA complementary to the mRNA of the flagellar gene, the siRNA complementary to the mRNA of the flagellar gene, and ribozymes complementary to the mRNA of the flagellar gene.

A microorganism in which the function of a flagellar gene has been suppressed can be selected by confirming its genomic sequence. Alternatively, a microorganism in which the function of a flagellar gene has been suppressed can be selected using the expression levels or activity of the flagellin protein as an indicator.

In the fermentation production of a useful substance using the modified microorganism of the present invention, the useful substance is not particularly limited. Examples thereof include proteins and polypeptides such as enzymes and bioactive factors useful for food, pharmaceuticals, cosmetics, detergents, textile treatment, and medical diagnostics. These include enzymes for various industries such as detergents, food, textiles, feed, chemicals, medical care, and diagnostics and bioactive peptides, and industrial enzymes are preferred. Industrial enzymes as classified based on the function include oxidoreductases, transferases, hydrolases, lyases, isomerases, and ligases/synthetases. Preferably, genes for hydrolases such as cellulase, α-amylase, protease, and lipase are included. Specific examples include α-amylase, particularly α-amylase derived from microorganisms, preferably bacteria of the genus *Bacillus.*

The integration of the gene encoding the above protein or polypeptide into the modified microorganism of the present invention can be performed by introducing into a host microorganism a recombinant plasmid harboring the gene encoding the above protein or polypeptide, using a general transformation method such as the competent cell transformation method, protoplast transformation method, or electroporation method. The integration of the gene can also be performed by directly integrating a DNA fragment, which contains the gene and is joined to an appropriate homologous region of the host microorganism genome, into the host microorganism genome via homologous recombination.

The fermentation production of a useful substance is performed by inoculating the modified microorganism of the present invention, into which a gene encoding the above protein or polypeptide has been integrated, into a medium containing an assimilable carbon source, a nitrogen source, and other essential components, culturing it by a conventional microbial culture method, and recovering and purifying the protein or polypeptide after the completion of the culture.

Here, the step of recovering the protein or polypeptide is a step of recovering the target substance (protein or polypeptide) from at least one of the cultured transformant and the culture of the transformant. After solid-liquid separation (centrifugation or coarse filtration), microfiltration (MF) is performed using a microfiltration membrane, and then purification and concentration are performed by ultrafiltration or the like as a case requires, to recover the target substance.

Microfiltration is a filtration method performed using a microfiltration membrane having an average pore diameter of 0.01 to 10 µm, preferably 0.01 µm to 0.5 µm, more preferably 0.01 µm to 0.1 µm, and even more preferably 0.05 to 0.1 µm, by pressure filtration, vacuum filtration, cross-flow filtration, centrifugal filtration, or the like. In the microfiltration, for example, an inorganic membrane such as a ceramic such as alumina titania, or zirconia, glass or metal membrane, or an organic membrane such as a cellulose acetate, nitrocellulose, aliphatic polyamide, polysulfone, polyolefin, polyacrylonitrile, polyethersulfone, polyvinyl chloride, polyvinyl alcohol, or fluoropolymer membrane, is used as the filtration membrane.

As shown in the Examples described below, when microfiltration (outlet pressure: 0.1 MPa, about 30 minutes) was performed on a culture solution obtained by culturing the modified microorganism of the present invention using a microfiltration membrane (polyethylene hollow fiber microfiltration membrane with an effective membrane area of 80 cm² and a pore diameter of 0.1 µm), the permeate flux (mL/cm²·min) is significantly improved compared to when the unmodified parent microorganism is used.

That is, in the fermentation production of a useful substance using the modified microorganism of the present invention, the improvement in filtration rate in the microfiltration step can be achieved. Here, the filtration rate can be calculated as the filtration membrane permeation flux per square centimeter per minute, and the improvement in the microfiltration rate of the present invention includes an improvement in the filtration rate by 110% or more, preferably 120% or more, and more preferably 150% or more, compared to when the unmodified parent microorganism is used. the clogging of the membrane in the microfiltration can be thereby reduced, and the time and the cost required for producing a useful substance can be reduced.

With respect to the embodiments described above, the following aspects are further disclosed in the present invention.
<1> A method for improving the filtration rate in the microfiltration step of the fermentation production of a useful substance using a microorganism, wherein a modified microorganism, in which the function of a flagellar gene of the microorganism is suppressed, is used as the microorganism.
<2> The method of <1>, wherein the suppression of the function of a flagellar gene is repression of the expression of the gene.
<3> The method of <1>, wherein the suppression of the function of a flagellar gene is a reduction in the expression level of the gene.
<4> The method of <3>, wherein the reduction in the expression level of the flagellar gene is achieved by deletion or inactivation of the gene.
<5> The method of any one of <1> to <4>, wherein the microorganism is a bacterium of the genus *Bacillus, Escherichia,* or *Brevibacillus.*
<6> The method of any one of <1> to <4>, wherein the microorganism is *Bacillus subtilis* or *Bacillus licheniformis.*
<7> The method of <6>, wherein the flagellar gene is a gene encoding a protein consisting of an amino acid sequence set forth in SEQ ID NO: 2 or 4, or an amino acid sequence having at least 90% or more identity thereto.
<8> The method of <6>, wherein the flagellar gene is a polynucleotide consisting of a nucleotide sequence set forth in SEQ ID NO: 1 or 3.
<9> The method according to any one of <6> to <8>, wherein the *Bacillus subtilis* is a mutant strain of *Bacillus subtilis* in which one or more sporulation-related genes or gene groups selected from the group consisting of the *sigE* gene, *sigF* gene, *spoIIE* gene, *spoIISB gene, sigG* gene, and gene groups included in the region from *spoIVCB* to *spoIIIC* have been deleted or inactivated.
<10> The method according to any one of <1> to <9>, wherein the microfiltration is performed using a microfiltration membrane having an average pore diameter of from 0.01 µm to 10 µm, preferably from 0.01 µm to 0.5 µm, and more preferably from 0.01 µm to 0.1 µm.

### Examples

Hereinafter, the present invention will be described in further detail with reference to Examples, however, the technical scope of the present invention is by no means limited to the following Examples.

### Example 1: Construction of hag gene-deleted strain

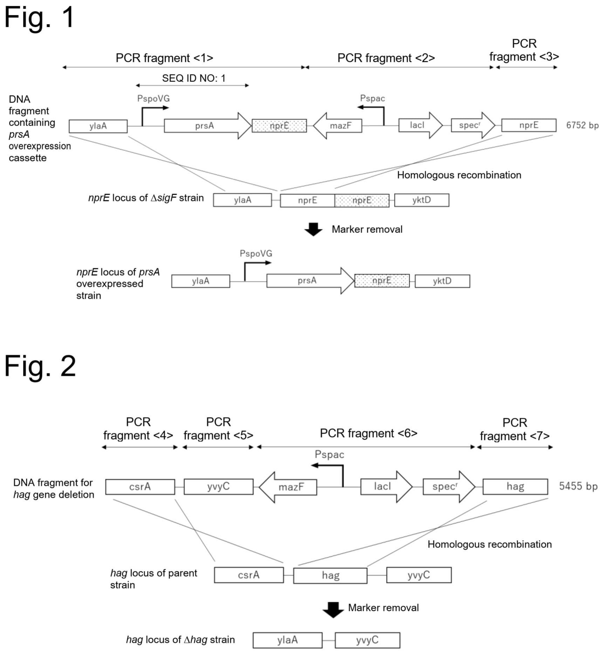

A strain of *Bacillus subtilis* 168, in which the *sigF* gene had been deleted (*ΔsigF* strain: JP-A-2003-47490), and into which an overexpression cassette of the *prsA* gene had been introduced, was used as the parent strain. The gene was introduced into the *nprE* locus of the *ΔsigF* strain by homologous recombination using a *prsA* gene overexpression cassette (SEQ ID NO: 11) (Figure 1). The DNA fragment containing the *prsA* overexpression cassette was constructed using the following method.

First, a PCR fragment <1> (primer prsA up F-prsA up R, template DNA: prsA-Ka strain genomic DNA (JP-B-4839144)), a PCR fragment <2> (primer prsA mid F-prsA mid R, template DNA: MazF cassette (Genet. Syst., 84(4):315-8, 2009)), and a PCR fragment <3> (primer prsA down F-prsA down R, template DNA: 168 strain genomic DNA) were constructed. Then, the PCR fragments <1> to <3> were joined by SOE-PCR (primer prsA final F-prsA final R). This gene transfer was performed in accordance with the marker-free deletion method developed by Morimoto et al. (Genet. Syst., 84(4):315-8, 2009). This *ΔsigF* strain in which the *prsA* gene was overexpressed was used as the parent strain in the present test.

The ORF sequence (SEQ ID NO: 1) of the hag gene in this parent strain was deleted by homologous recombination using a hag gene deletion cassette (Figure 2). The DNA fragment for *hag* gene deletion was constructed using the following method. First, a PCR fragment <4> (primer hag up F-hag up R, template DNA: 168 strain genomic DNA), a PCR fragment <5> (primer hag mid F-hag mid R, template DNA: 168 strain genomic DNA), a PCR fragment <6> (primer mazF F-mazF R, template DNA: MazF cassette (Genet. Syst., 84(4):315-8, 2009)), and a PCR fragment <7> (primer hag down F-hag down R, template DNA: 168 strain genomic DNA) were constructed. Then, the PCR fragments <4> to <7> were joined by SOE-PCR (primer hag final F-hag final R). This gene transfer was performed in accordance with the marker-free deletion method developed by Morimoto et al. (Genet. Syst., 84(4):315-8, 2009). Hereinafter, this hag gene-deleted strain is referred to as the *Δhag* strain.

**[Table 2]**

| Primer | 5'-sequence | SEQ ID NO |
|---|---|---|
| prsA up F | TTTCAAATGAGTAGAAATAATGCACATC | 12 |
| prsA up R | TGGGTAGGATCCGCGCCGCTGTTTGTGTGTACAC CGCCA | 13 |
| prsA mid F | ACACACAAACAGCGGCGCGGATCCTACCCAATCA GTACGTTAA | 14 |
| prsA mid R | ACACACAAACAGCGGCGCGGATCCTACCCAATCA GTACGTTAA | 15 |
| prsA down F | ACACACAAACAGCGGCGCGG | 16 |
| prsA down R | CTTTTGTTTCTATCGCTTTTAATTCG | 17 |
| prsA final F | GAAATGTGCTCCCCGACAAAATTG | 18 |
| prsA final R | TTGGCCGCTCCGTTAGAAACAGC | 19 |
| prsA fw | TTTCAAATGAGTAGAAATAATGCACATC | 20 |
| prsA rv | TTCCAGGCTGCTTCAACTTTAG | 21 |
| hag up F | TCATTCATACGAAGTACCATGGCCAAATGA | 22 |
| hag up R | CGCCAAGGTCTTTTTTAAAATGTTTTGTTCCTCCC TGAAT | 23 |
| hag mid F | ATTCAGGGAGGAACAAAACATTTTAAAAAAGACC TTGGCG | 24 |
| hag mid R | CTGATTGGGTAGGATCCGCGCAGTTTTGCTCTGAT ACGTG | 25 |
| mazF F | CGCGGATCCTACCCAATCAGTACG | 26 |
| mazF R | CGACAGCGGAATTGACTCAAGC | 27 |
| hag down F | GAGTCAATTCCGCTGTCGTCTTCAGGTCTTCGCAT CAA | 28 |
| hag down R | AAGTACGTTTTGCGGCTGTTGGTTTGCTTG | 29 |
| hag final F | AATCATTCTTTTTGAAAGCGGGATTCCAGG | 30 |
| hag final R | AGCAAGCATAGCTTGAGAAGCCTGAGAAAG | 31 |

### Example 2: Introduction of amylase expression vector

An amylase expression plasmid (pHY-YR288 (Patent Application No. 2021-112712)) was introduced into the parent strain and the *Δhag* strain obtained in Example 1 by the protoplast transformation method.

### Example 3: Culture evaluation

Culture evaluation was conducted using a 2-L jar fermenter in accordance with the following method. The strain containing the amylase expression vector obtained in Example 2 was subjected to a shaking culture overnight at 30°C and 180 rpm in 30 mL of LB medium supplemented with 10 ppm tetracycline hydrochloride in a 500-mL baffled erlenmeyer flask. 25 mL of this culture solution were collected and inoculated into 1.2 L of 2x L-maltose medium (2% soytone, 1% yeast extract, 1% NaCl, 7.5% maltose, 7.5 ppm manganese tetra/pentahydrate, and an antifoaming agent), followed by culturing at 30°C, 850 rpm, and 0.04 MPa for 3 days.

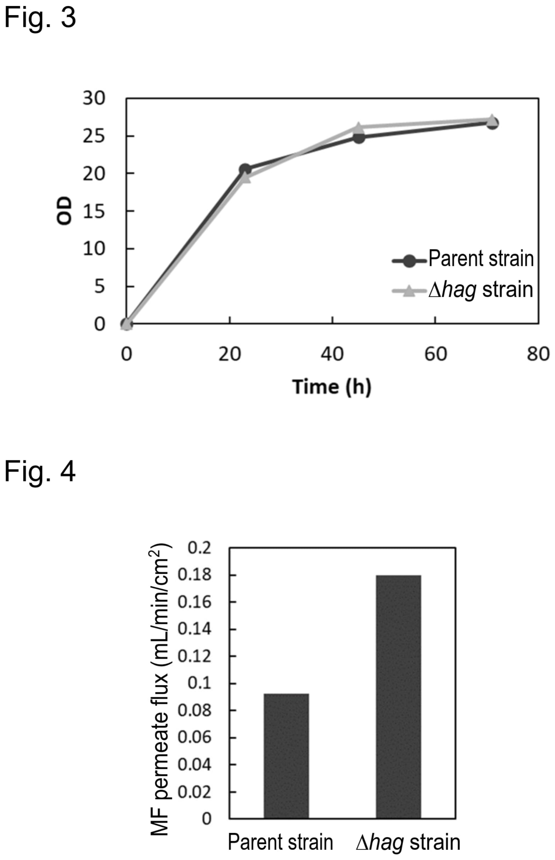

The cell density of the culture solution was measured at a wavelength of 600 nm using a Hitachi spectrophotometer (U-2900) (Hitachi High-Tech Corporation). The cell density of the *Δhag* strain was equivalent to that of the parent strain, indicating that deletion of the *hag* gene does not affect cell growth (Figure 3).

### Example 4: Measurement of MF permeate flux

Microfiltration (MF) was conducted using the culture solution obtained in Example 3 by the following method. Samples of the culture solution on day 3 (OD600 was about 27 for both the parent strain and the *Δhag* strain) were used. Microfiltration (MF) was performed using, as the MF module, a polyethylene hollow fiber microfiltration membrane microsa PSP-013 (Asahi Kasei Corporation) having an effective membrane area of 80 cm² and a pore diameter of 0.1 µm. The pump used for the microfiltration system was an EYELA RP-1100 (Tokyo Rikakikai Co., Ltd.), with a pump rotation speed set at 100 rpm. The MF module stand used was a microsa PX-02001, and the valve was adjusted to obtain an outlet pressure of 0.1 MPa. Filtration was performed under these conditions for about 30 minutes until the permeate flux remained constant, and the permeate flux at that time was evaluated. The results are shown in Figure 4. These results indicate that the deletion of the *hag* gene significantly improves the MF permeate flux in culture solutions with equivalent cell densities.

## Claims

1. A method for improving the filtration rate in the microfiltration step of the fermentation production of a useful substance using a microorganism, wherein a modified microorganism, in which the function of a flagellar gene of the microorganism is suppressed, is used as the microorganism.

2. The method according to claim 1, wherein the suppression of the function of a flagellar gene is repression of the expression of the gene.

3. The method according to claim 1, wherein the suppression of the function of a flagellar gene is a reduction of the expression level of the gene.

4. The method according to claim 3, wherein the reduction in the expression level of the flagellar gene is achieved by deletion or inactivation of the gene.

5. The method according to any one of claims 1 to 4, wherein the microorganism is a bacterium of the genus *Bacillus, Escherichia,* or *Brevibacillus.*

6. The method according to any one of claims 1 to 4, wherein the microorganism is *Bacillus subtilis* or *Bacillus licheniformis.*

7. The method according to claim 6, wherein the flagellar gene is a gene encoding a protein consisting of an amino acid sequence set forth in SEQ ID NO: 2 or 4, or an amino acid sequence having at least 90% or more identity thereto.

8. The method according to claim 6 or 7, wherein the *Bacillus subtilis* is a mutant strain of *Bacillus subtilis* in which one or more sporulation-related genes or gene groups selected from the group consisting of the *sigE* gene, *sigF* gene, *spoIIE* gene, *spoIISB gene, sigG* gene, and gene groups included in the region from *spoIVCB* to *spoIIIC* have been deleted or inactivated.

9. The method according to any one of claims 1 to 8, wherein the microfiltration is performed using a microfiltration membrane having an average pore diameter of from 0.01 µm to 10 µm.
